# EUROPÄISCHE PATENTANMELDUNG

(11) **EP 4 563 562 A1**
(43) Veröffentlichungstag der Anmeldung: **04.06.2025**
(21) Anmeldenummer: 24213335.3
(22) Anmeldetag: 15.11.2024
(51) Int. Cl.: C07C 7/04, C07C 7/00, C07C 11/08, B01D 1/28, B01D 3/00, B01D 3/14

(54) **ENERGIEEFFIZIENTES VERFAHREN ZUR ABTRENNUNG VON 1-BUTEN AUS EINEM KOHLENWASSERSTOFFSTROM**

(30) Priorität: 29.11.2023 EP 23213045
(71) Anmelder: Evonik Oxeno GmbH & Co. KG, 45772 Marl (DE)
(72) Erfinder: Paul, Niklas, 45770 Marl (DE); Rix, Armin Matthias, 45770 Marl (DE); Schröder, Moritz, 48153 Münster (DE); Six, Tanita Valèrie, 44309 Dortmund (DE); Steenweg, Tanja, 44139 Dortmund (DE); Mellaerts, Wim, 2940 Hoevenen (BE); Knossalla, Johannes, 48351 Everswinkel (DE); Peitz, Stephan, 45739 Oer-Erkenschwick (DE); Stochniol, Guido, 45721 Haltern am See (DE); Laiblin, Tobias, 2930 Brasschaat (BE)
(74) Vertreter: Evonik Patent Association

(57) **Zusammenfassung**

Gegenstand der Erfindung ist ein Verfahren zur Abtrennung von 1-Buten aus einem Kohlenwasserstoffstrom, der zumindest 1-Buten, 2-Buten, n-Butan und Isobutan enthält, in einer Abtrenneinheit, die mindestens zwei Destillationskolonnen DK1 und DK2 umfasst, wobei die Kondensationswärme genutzt wird, um Energiekosten einzusparen und CO₂-Emission zu verringern.

## Beschreibung

Die vorliegende Erfindung betrifft ein Verfahren zur Abtrennung von 1-Buten aus einem C4-Kohlenwasserstoffstrom, der zumindest 1-Buten, 2-Buten, n-Butan und Isobutan enthält, in einer Abtrenneinheit, die mindestens zwei Destillationskolonnen DK1 und DK2 umfasst, wobei die Kondensationswärme nutzbar gemacht wird, um Energiekosten einzusparen und CO₂-Emission zu verringern.

1-Buten kann in großen Mengen aus technischen C4-Kohlenwasserstoffströmen, beispielsweise dem C4-Schnitt aus Steamcrackern oder FCC-Einheiten, gewonnen werden. Diese C4-Kohlenwasserstoffströme bestehen im Wesentlichen aus Butadien, den Monoolefinen Isobuten, 1-Buten und den beiden 2-Butenen (cis- und trans-2-Buten) sowie den gesättigten Kohlenwasserstoffen Isobutan und n-Butan. Wegen der geringen Siedepunktsunterschiede der Inhaltsstoffe, deren geringen Trennfaktoren und der Ausbildung von Azeotropen ist eine ausschließlich destillative Aufarbeitung von C4-Kohlenwasserstoffströmen schwierig und nicht wirtschaftlich.

Üblicherweise wird deshalb zunächst das Butadien mittels Extraktivdestillation abgetrennt oder selektiv zu Butenen hydriert. Zurück bleibt jeweils ein C4-Kohlenwasserstoffstrom (sogenanntes Raffinat 1), der neben den gesättigten Kohlenwasserstoffen n-Butan und Isobutan die Olefine Isobuten, 1-Buten und 2-Butene enthält, während das Butadien maximal in geringen Mengen vorhanden ist.

Da die Siedepunkte von 1-Buten und Isobuten eng beieinanderliegen, kann aus entsprechenden C4-Kohlenwasserstoffströmen in der Regel kein 1-Buten über einfache Destillation wirtschaftlich abgetrennt werden. Das Isobuten wird deshalb möglichst weitgehend entfernt, beispielsweise über eine MTBE- oder ETBE-Synthese. Durch die Entfernung des Isobutens entsteht ein C4-Kohlenwasserstoffstrom (sogenanntes Raffinat 2), der die linearen Butene (1- und 2-Butene) und die gesättigten Kohlenwasserstoffe Isobutan und n-Butan enthält.

Die Abtrennung von 1-Buten aus derartigen C4-Kohlenwasserstoffströmen ist möglich und wird in der chemischen Industrie eingesetzt. Die Abtrennung erfolgt dabei in einer Destillationseinheit, die mindestens zwei Destillationskolonnen umfasst. In der ersten Destillationskolonne werden Isobutan und 1-Buten am Kopf anfallen und zur zweiten Destillationskolonne geleitet. In der zweiten Destillationskolonne werden dann Isobutan und 1-Buten voneinander getrennt. Ein solches Verfahren wird beispielsweise in der DE 10 2005 062 700 A1 offenbart.

Die für die Auftrennung des C4-Kohlenwasserstoffstroms notwendige Energie wird bei den bekannten Verfahren üblicherweise über Heizdampf in den Sumpf der beiden Destillationskolonnen eingebracht. Zwar steht an chemischen Produktionsstandorten Heizdampf in aller Regel zur Verfügung. In den für die betreffenden Trennaufgaben benötigten Mengen, bedeutet der Einsatz von Heizdampf einen nicht zu unterschätzenden Kostenfaktor. Zudem ist die Rückführung des gebrauchten Heizdampfs logistisch nicht immer einfach, da der Dampf nur innerhalb gewisser Spezifikationen (Druck, Temperatur, o. ä.) zurückgefahren werden dann. Darüber hinaus wird bei der Erzeugung von Heizdampf eine große Menge an CO₂ erzeugt.

Die Aufgabe der vorliegenden Erfindung bestand deshalb darin ein Verfahren bereitzustellen, bei dem Energie und CO₂ im Vergleich zu den bekannten Verfahren eingespart werden können und das in bestehende Anlagen integriert werden kann.

Diese Aufgabe wird durch die in Anspruch 1 vorgeschlagene Ausführung des Verfahrens gelöst. Bevorzugte Ausführungsformen sind in den Unteransprüchen angegeben. Das erfindungsgemäße Verfahren ist ein Verfahren zur Abtrennung von 1-Buten aus einem Raffinat-2-Strom, der zumindest 1-Buten, 2-Buten, n-Butan und Isobutan enthält, in einer Abtrenneinheit, die mindestens zwei Destillationskolonnen DK1 und DK2 umfasst, wobei
die erste Destillationskolonne DK1 mindestens zwei Sumpfverdampfer SV1a und SV1b und die zweite Destillationskolonne DK2 mindestens einen Sumpfverdampfer SV2a aufweist;
die Sumpfverdampfer SV1a und SV1b jeweils mit einem Strom gespeist werden, der am unteren Ende der DK1 entnommen wird und nach Durchlaufen des jeweiligen Sumpfverdampfers wieder zur DK1 geführt wird;
der Sumpfverdampfer SV2a mit einem Strom gespeist wird, der am unteren Ende der DK2 entnommen wird und nach Durchlaufen des jeweiligen Sumpfverdampfers wieder zur DK2 geführt wird; wobei das Verfahren die folgenden Schritte umfasst
   (a) der Raffinat-2-Strom zur ersten Destillationskolonne DK1 geleitet wird und in der DK1 in mindestens einen Brüdenstrom BS1, der zumindest 1-Buten und Isobutan umfasst und am Kopf der DK1 entnommen wird, und in mindestens einen Sumpfstrom, der zumindest 1-Buten und 2-Buten umfasst und am Sumpf der DK1 entnommen wird, aufgetrennt wird;
   (b) der Brüdenstrom in mindestens zwei Teilströme BS1a und BS1b aufgetrennt wird;
   (c) ein erster Teil BS1a des Brüdenstroms BS1 verdichtet wird, wodurch ein gegenüber dem Brüdenstrom BS1a verdichteter Strom VB1 entsteht;
   (d) Energie vom verdichteten Strom VB1 auf den Strom im Sumpfverdampfer SV1a übertragen wird;
   (e) ein von BS1a verschiedener Teil BS1b des Brüdenstroms BS1 zum Sumpfverdampfer SV2a geführt wird und dort Energie von BS1b auf den Strom im Sumpfverdampfer SV2a übertragen wird;
   (f) die Ströme VB1 und BS1b zumindest teilweise zur zweiten Destillationskolonne DK2 geleitet werden und in der DK2 in mindestens einen Brüdenstrom BS2, der zumindest Isobutan umfasst und am Kopf der DK2 entnommen wird, und in mindestens einen Produktstrom, der zumindest 1-Buten umfasst und am Sumpf der DK2 entnommen wird, aufgetrennt wird;
   (g) der Brüdenstrom BS2 zumindest teilweise verdichtet wird, wodurch ein gegenüber dem Brüdenstrom BS2 verdichteter Strom VB2 entsteht; und
   (h) Energie vom verdichteten Strom VB2 auf den Strom im Sumpfverdampfer SV1b übertragen wird.

Ein Vorteil des erfindungsgemäßen Verfahrens ist die doppelte Wärmeintegration über die verdichteten Ströme VB1 und VB2, von denen Energie in den Sumpfverdampfern SV1a und SV1b auf die jeweiligen Ströme übertragen und so Energie in den Sumpf eingetragen wird. Die Energieübertragung in den Sumpfverdampfern SV1a und SV1b in der ersten Destillationskolonne DK1 hat zur Folge, dass weniger oder gar kein Heizdampf zur Beheizung der Destillationskolonne DK1 eingesetzt werden muss. Es kann also eine (fast) vollständige Verstromung des energieintensiven Verfahrens erzielt werden, was wiederum den Einsatz von grünem Strom ermöglicht. Dadurch werden erhebliche Mengen an Energiekosten und CO₂ eingespart.

Der Ausgangsstrom, aus dem das 1-Buten abgetrennt werden soll, ist nach der vorliegenden Erfindung ein Raffinat-2-Strom, der zumindest 1-Buten, 2-Buten, n-Butan und Isobutan enthält. Entsprechende Ströme sind auf dem Markt verfügbar, beispielsweise als C4-Schnitt aus Steamcrackern oder FCC-Einheiten. Es wurde in der Einleitung bereits erwähnt, dass Raffinat 2 dadurch entsteht, dass mehrfach ungesättigte C4-Kohlenwasserstoffe, insbesondere Butadien, und Isobuten aus dem Strom entfernt werden. Eine vollständige Entfernung ist aus wirtschaftlichen und technischen Gründen in vielen Fällen nicht möglich. Die Mengen an mehrfach ungesättigten C4-Kohlenwasserstoffen, insbesondere Butadien, und Isobuten sollten jedoch möglichst gering sein.

Vorzugsweise enthält das eingesetzte Raffinat 2 weniger als 1000 ppm, vorzugsweise weniger als 500 ppm Isobuten. Sind höhere Mengen an Isobuten im Ausgangstrom vorhanden, könnte zwischen die beiden Destillationskolonnen DK1 und DK2 eine MTBE- oder ETBE-Synthese (Methyl-tert.-butyl-ether = MTBE / Ethyl-tert.-butyl-ether = ETBE) erfolgen, um das Isobuten mit Methanol (für MTBE) oder Ethanol (ETBE) umzusetzen und anschließend das MTBE oder ETBE abzutrennen. So kann die Konzentration an Isobuten vor der zweiten Destillationskolonne DK2 stark reduziert werden. Isobuten würde in der zweiten Destillationskolonne nämlich im Sumpf und damit im 1-Buten anfallen.

Weiterhin bevorzugt enthält das im Verfahren der vorliegenden Erfindung eingesetzte Raffinat 2 weniger als 4 Gew.-% mehrfach ungesättigte C4-Kohlenwasserstoffe. In einer besonders bevorzugten Ausführungsform sollte die die Konzentration der mehrfach ungesättigten C4-Kohlenwasserstoffe weniger als 500 ppm betragen. Sollten die Ströme höhere Mengen an Butadien enthalten, könnte vorher eine Selektivhydrierung durchgeführt werden, bei der das Butadien zu Butenen und/oder Butanen umgesetzt wird. Entsprechende Verfahren sind dem Fachmann beispielsweise in der EP 3 680 224 A1 beschrieben.

Der eingesetzte Raffinat-2-Strom kann darüber hinaus gewisse Mengen an Wasser enthalten, insbesondere in einer Menge von 150 bis 4000 ppm. Es ist bevorzugt, dass das Wasser durch das hier beschriebene Verfahren zumindest teilweise abgetrennt wird. Das Wasser wird sich dabei in jeder der beiden Destillationskolonnen DK1 und DK2 im jeweiligen Brüdenstrom BS1 und BS2 anreichern und nach Kondensation als zweite flüssige Phase anfallen, die über Euter in den Destillatbehältern der DK1 und/oder der DK2 abgetrennt werden können. Die Sumpfprodukte der DK1 und der DK2 zeichnen sich durch sehr niedrige Gehalte an Butadien und Wasser, bevorzugt je unter 100 ppm, besonders bevorzugt unter 5 ppm aus.

Das erfindungsgemäße Verfahren wird in einer Abtrenneinheit durchgeführt, die mindestens die beiden Destillationskolonnen DK1 und DK2 umfasst. DK1 ist die erste Destillationskolonne und weist mindestens zwei Sumpfverdampfer SV1a und SV1b auf. DK2 ist die zweite Destillationskolonne und weist mindestens einen Sumpfverdampfer SV2a auf. In einer bevorzugten Ausführungsform weist die Destillationskolonne nur die beiden Sumpfverdampfer SV1a und SV1b auf. Es ist zudem bevorzugt, dass die Destillationskolonne DK2 nur den einen Sumpfverdampfer SV2a aufweist. Die Drücke in den beiden Destillationskolonnen DK1 und DK2 sollten insbesondere so gewählt werden müssen, dass Wärme in den Sumpfverdampfern übertragen werden kann. Die Begriffe "erste Destillationskolonne", "Destillationskolonne DK1" und "DK1" sind im Rahmen der vorliegenden Erfindung als synonym zu verstehen. Ebenso sind die sie Begriffe "zweite Destillationskolonne", "Destillationskolonne DK2" und "DK2" im Rahmen der vorliegenden Erfindung als synonym zu verstehen.

Über die Sumpfverdampfer SV1a und SV1b wird die für die Trennaufgabe notwendige Energie in die erste Destillationskolonne DK1 eingebracht. Die Sumpfverdampfer SV1a und SV1b werden beide jeweils mit einem Strom gespeist, der am unteren Ende der DK1 entnommen und nach Durchlaufen des jeweiligen Sumpfverdampfers wieder zur DK1 geführt wird. Der jeweilige Strom wird beim Durchlaufen des Sumpfverdampfers SV1a oder SV1b erhitzt. Die beiden Ströme können entweder unabhängig voneinander, d. h. an zwei voneinander unterschiedlichen Stellen des Sumpfes der DK1 entnommen werden. Es kann aber auch nur ein Strom entnommen werden, der danach in die beiden Ströme getrennt wird, ggf. mit einer Split-Regelung, über die die Massenströme der beiden Ströme anhand eines vorher festgelegten Parameters eingestellt werden. Der Zulauf der beiden Ströme aus den beiden Sumpfverdampfern SV1a und SV1b sind insbesondere an unterschiedlichen Stellen des Sumpfes, d. h. die beiden Ströme werden nach Durchlaufen des Sumpfverdampfer nicht vor Eintritt in die DK2 vermischt.

Vergleichbares gilt für den Sumpfverdampfer SV2a der zweiten Destillationskolonne DK2, durch den für die Trennaufgabe notwendige Energie eingetragen wird. Der Sumpfverdampfer SV2a wird dazu mit einem Strom gespeist wird, der am unteren Ende der DK2 entnommen und nach Durchlaufen des jeweiligen Sumpfverdampfers wieder zur DK2 geführt wird. Der Strom wird beim Durchlaufen des Sumpfverdampfers SV2a erhitzt und verdampft dabei zumindest teilweise.

Als "Sumpfverdampfer" werden erfindungsgemäß Verdampfer bezeichnet, die den Sumpf der jeweiligen Destillationskolonne beheizen. Ein derartiger Sumpfverdampfer ist üblicherweise außerhalb der jeweiligen Destillationskolonne angeordnet. Da in Sumpfverdampfern Energie, insbesondere Wärme, von einem Strom auf einen anderen übertragen wird, sind sie Wärmeüberträger. Über einen Abzug aus dem Sumpf der Destillationskolonne wird der zu verdampfende Strom abgezogen und dem Sumpfverdampfer zugeführt. Über mindestens einen Zulauf wird der verdampfte Strom gegebenenfalls mit einem Restanteil Flüssigkeit wieder in die jeweilige Destillationskolonne im Bereich des Sumpfs zurückgeführt.

Geeignete Verdampfer, die als Sumpfverdampfer eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Als Wärmeübertrager für den Verdampfer wird bei Naturumlaufverdampfern und Zwangsumlaufverdampfern üblicherweise ein Rohrbündel oder Plattenapparat eingesetzt. Neben den genannten kann aber auch jede beliebige andere, dem Fachmann bekannte Verdampferbauart, die sich zum Einsatz an einer Destillationskolonne eignet, eingesetzt werden.

Das Raffinat 2, das zur ersten Destillationskolonne DK1 geleitet wird, wird in der Destillationskolonne DK1 in mindestens zwei Ströme aufgetrennt, d. h. in mindestens einen Brüdenstrom BS1, der zumindest 1-Buten und Isobutan umfasst und am Kopf der DK1 entnommen wird, und in mindestens einen Sumpfstrom, der zumindest 1-Buten und 2-Buten umfasst und am Sumpf der DK1 entnommen wird. Dieser Sumpfstrom kann zu einer Oligomerisierung (nicht gezeigt) geführt werden. Der Brüdenstrom BS1 kann am Kopf der Destillationskolonne auch in Form von mehreren Teilströmen BS1n, wobei n eine ganze Zahl und gleich der Anzahl der Teilströme ist, entnommen werden. Gleiches gilt auch für den Sumpfstrom. Im Sumpf der ersten Destillationskolonne DK1 liegt vorzugsweise eine Temperatur im Bereich von 40 bis 110 °C, vorzugsweise 50 bis 100 °C vor.

Grundsätzlich kann der Raffinat-2-Strom über eine oder mehrere Zulaufstellen in die erste Destillationskolonne DK1 geleitet werden. Sind mehrere Zulaufstellen für den Raffinat-2-Strom vorhanden, werden demnach mehrere voneinander getrennte Ströme in die Destillationskolonne geleitet. In den Ausführungsformen der vorliegenden Erfindung, in denen das der Raffinat-2-Strom als zwei oder mehrere voneinander getrennte Ströme in die Destillationskolonne DK1 geleitet wird, ist es vorteilhaft, wenn die Zulaufstellen der einzelnen Ströme im Wesentlichen auf der gleichen Höhe an der Destillationskolonne DK1 liegen.

Im Folgenden werden der Druck und die Temperatur des Brüdenstroms BS1 angegeben. Dies bezieht sich insbesondere auf den Druck und die Temperatur des mindestens einen Brüdenstroms BS1, wenn er der Destillationskolonne DK1 entnommen wird. Der Druck des Brüdenstroms BS1 liegt insbesondere im Bereich von 6 bis 15 bar absolut, vorzugsweise im Bereich von 7,5 bis 13 bar absolut. Die Temperatur des Brüdenstroms BS1 liegt insbesondere im Bereich von 45 °C bis 120 °C, bevorzugt im Bereich von 48 °C bis 100 °C, weiterhin bevorzugt im Bereich von 50 °C bis 90 °C, weiterhin bevorzugt im Bereich von 55 °C bis 80 °C, besonders bevorzugt im Bereich von 60 °C bis 80 °C.

Als Destillationskolonne DK1 für die Auftrennung des Raffinat-2-Stroms kann jede beliebige, dem Fachmann bekannte Destillationskolonne eingesetzt werden. Bevorzugt enthält die Destillationskolonne DK1 Einbauten. Geeignete Einbauten sind zum Beispiel Böden, unstrukturierte Packungen (Füllkörper) oder strukturierte Packungen. Als Böden werden üblicherweise Glockenböden, Siebböden, Ventilböden, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel oder Intalox^{®}-Sättel verwendet. Strukturierte Packungen werden zum Beispiel unter dem Handelsnamen Mellapack^{®} der Firma Sulzer vertrieben. Neben den genannten Einbauten sind dem Fachmann weitere geeignete Einbauten bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Destillationskolonne DK1 möglichst gering bleibt und somit die mechanische Leistung des Verdichters und die Temperatur des zu verdampfenden Raffinat-2-Stroms gering bleibt.

In einer besonders bevorzugten Ausführungsform der vorliegenden Erfindung umfasst die Destillationskolonne DK1 eine Vielzahl von Böden, vorzugsweise zwischen 150 und 300 Böden, weiterhin bevorzugt zwischen 170 und 220 Böden.

Die Entnahme von mindestens einem Brüdenstroms BS1, der zumindest 1-Buten und Isobutan umfasst, am Kopf der Destillationskolonne DK1 bedeutet im Rahmen der vorliegenden Erfindung insbesondere, dass der mindestens eine Brüdenstrom BS1 als Kopfstrom oder als Seitenabzug oberhalb der Einbauten in der Destillationskolonne DK1 entnommen wird.

Die Entnahme des mindestens einen Sumpfstroms, der zumindest 1-Buten und 2-Buten umfasst, am Sumpf der Destillationskolonne DK1 bedeutet im Rahmen der vorliegenden Erfindung insbesondere, dass der mindestens eine Sumpfstrom direkt am Sumpf oder am unteren Boden der Destillationskolonne DK1 entnommen wird.

Die Destillationskolonne DK1 wird weiterhin mit Rücklauf, vorzugsweise mit Rücklauf betrieben. Rücklauf bedeutet, dass der am oberen Ende der Destillationskolonne DK1 entnommene Brüdenstrom BS1 mindestens teilweise wieder der Destillationskolonne DK1 zugeführt wird. In den Fällen, in denen ein solcher Rücklauf eingestellt wird, beträgt das Rücklaufverhältnis dabei bevorzugt 2 bis 30, bevorzugter 5 bis 20, besonders bevorzugt 8 bis 15.

Ein Rücklauf kann dadurch eingestellt werden, dass am Kopf der Destillationskolonne DK1 ein Kondensator angebracht wird. In dem Kondensator wird Brüdenstrom BS1 teilweise kondensiert und der Destillationskolonne DK1 wieder zugeführt. Der Brüdenstrom oder ein Teil davon kann auch erst nach Verdichtung und Entspannung als Rücklauf auf die Destillationskolonne gegeben werden. Unter einem Rücklaufverhältnis wird allgemein und im Sinne dieser Erfindung das Verhältnis aus dem Anteil des aus der Kolonne entnommenen Massenstroms (kg/h), der wieder auf die Kolonne in flüssiger Form zurückgeführt wird (Rücklauf) zu dem Anteil dieses Massenstroms (kg/h), der in flüssiger Form oder gasförmiger Form von der jeweiligen Kolonne abgeführt wird, verstanden.

Nach der Entnahme des Brüdenstroms BS1 wird der Brüdenstrom BS1 in Schritt (b) in mindestens zwei Teilströme BS1a und BS1b aufgetrennt. Die Auftrennung kann grundsätzlich auf bekannte Weise, beispielsweise durch einen Splitter (Regelung an einem Verdichter und/oder einem Stellventil) erfolgen. Denkbar wäre hier auch eine Regelung, über die die Massenströme von BS1a und BS1b in Abhängigkeit eines bestimmten Parameters eingestellt werden.

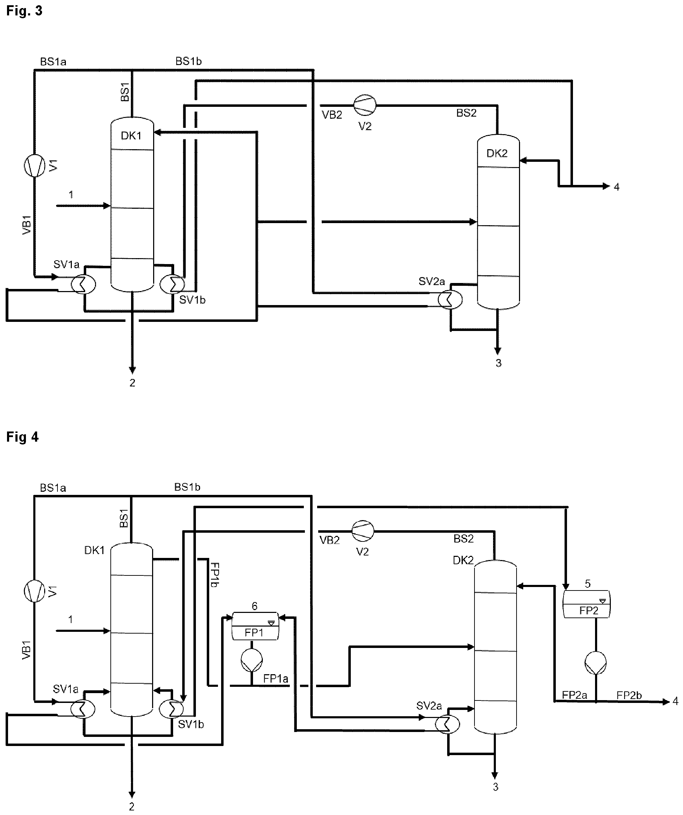

Anschließend wird in Schritt (c) ein erster Teil BS1a des Brüdenstroms BS1 verdichtet, wodurch ein gegenüber dem Brüdenteilstrom BS1a verdichteter Strom VB1 entsteht. Der Strom BS1a weist dabei in der Regel den gleichen Druck auf wie der Strom BS1, wenn er der Destillationskolonne DK1 entnommen wird. Es kann vorteilhaft sein den Strom BS1a vor dem Verdichten zu erwärmen, damit bei der Verdichtung kein zweiphasiges Gemisch entsteht. Die Erwärmung kann mittels interner (s. WT-1 in Fig. 3 und 4 der vorliegenden Anmeldung) oder externer Wärmequellen durchgeführt werden. Der Druck von VB1 ist nach dem Verdichten höher als der Druck von BS1a. Der genaue Wert für den Druck von VB1 kann in Abhängigkeit der Anforderungen bei der nachfolgenden Energieübertragung vom Fachmann eingestellt werden, solange die Bedingung Druck VB1 > Druck BS1a erfüllt ist. Der Quotient aus Druck VB1 / Druck BS1a (Drücke jeweils in bar abs.) liegt bevorzugt im Bereich von 1,1 bis 10, bevorzugter 1,2 bis 8, bevorzugter 1,25 bis 7, am bevorzugtesten 1,3 bis 6.

Die Temperatur des Teilstroms VB1 ist vorzugsweise höher als die Temperatur des Brüdenteilstroms BS1a, und der Quotient aus Temperatur VB1 / Temperatur BS1a (Temperatur jeweils in K) liegt bevorzugt im Bereich von 1,03 bis 10, bevorzugter 1,04 bis 9, bevorzugter 1,05 bis 8, bevorzugter 1,06 bis 7, bevorzugter 1,07 bis 6, am bevorzugtesten 1,08 bis 5.

Das Verdichten des mindestens einen Teils des Brüdenstroms BS1a in Schritt (c) kann auf jede beliebige, dem Fachmann bekannte Art erfolgen. So kann die Verdichtung zum Beispiel mechanisch und einstufig oder mehrstufig durchgeführt werden. Einstufig bedeutet in diesem Zusammenhang, dass eine Verdichtung von einem auf ein anderes Druckniveau stattfindet. Mehrstufig bedeutet, dass erst auf ein Druckniveau X und dann von X auf das Druckniveau Y verdichtet wird. Bei einer mehrstufigen Verdichtung können mehrere Verdichter gleicher Bauart oder Verdichter unterschiedlicher Bauart eingesetzt werden. Eine mehrstufige Verdichtung kann mit einer oder mehreren Verdichtermaschinen erfolgen. Der Einsatz einer einstufigen Verdichtung oder einer mehrstufigen Verdichtung ist vom Verdichtungsverhältnis und somit davon abhängig, auf welchen Druck der Brüdenteilstrom BS1a verdichtet werden soll.

Als Verdichter im erfindungsgemäßen Verfahren, insbesondere zum Verdichten der Brüdenströme BS1a zu VB1, eignet sich jeder beliebige, dem Fachmann bekannte Verdichter, bevorzugt mechanische Verdichter, mit dem sich Gasströme verdichten lassen. Geeignete Verdichter sind zum Beispiel ein- oder mehrstufige Getriebeturboverdichter, Kolbenverdichter, Schraubenverdichter, Zentrifugalverdichter oder Axialverdichter.

Im Schritt (d) des erfindungsgemäßen Verfahrens wird Energie vom verdichteten Strom VB1 auf den Strom im Sumpfverdampfer SV1a übertragen. Durch den Schritt (d) sinkt die Energie von VB1, so dass VB1 insbesondere mindestens teilweise kondensiert. Die Phrase Übertragung von Energie bedeutet erfindungsgemäß insbesondere Beheizung, also Übertragung von Energie in Form von Wärme.

Die Übertragung von Energie von VB1 auf den Strom im Sumpfverdampfer SV1a, bevorzugt die Beheizung des Stroms im Sumpfverdampfer SV1a durch VB1 erfolgt bevorzugt direkt. Direkte Übertragung bedeutet, dass VB1 und der Strom in SV1a sich zwar nicht direkt kontaktieren, aber dass Energie, insbesondere Wärme, von VB1 auf den Strom im SV1a übergeht, ohne dass ein zusätzliches Wärmeträgermedium vorhanden ist. Als Sumpfverdampfer SV1a können die dem Fachmann geläufigen Wärmeüberträger bzw. Wärmetauscher, insbesondere Verdampfer, eingesetzt werden.

Der Schritt (d) des erfindungsgemäßen Verfahrens kann in einer bevorzugten Ausführungsform zu einem besonderen Vorteil führen. Dabei dissipiert die bei der Verdichtung des Brüdenstrom BS1a zum verdichteten Brüdenstrom VB1 erhaltene überschüssige Energie nicht ungenutzt, sondern wird in der Destillation DK2 eingesetzt. Dies erfolgt dergestalt, dass zuerst die Verdichtung von BS1a zu VB1 so erfolgt, dass VB1 über das in dem SV1a erforderliche Maß hinaus verdichtet wird. Die Kondensationswärme, die bei der zusätzlichen Verdichtung erhalten wird, kann über den Strom VB1 in die Kolonne DK2 eingespeist werden. Die erforderliche zusätzliche Verdichterleistung ist in der Regel geringer als die dadurch eingesparte Heizdampfleistung.

Im Schritt (e) des erfindungsgemäßen Verfahrens wird mindestens ein von BS1a verschiedener Teil BS1b des Brüdenstroms BS1 zum Sumpfverdampfer SV2a geführt wird und dort im Sumpfverdampfer SV2a Energie von BS1b auf den im Sumpfverdampfer vorhandenen Strom übertragen Durch den Schritt (e) sinkt die Energie von BS1b, so dass BS1b insbesondere mindestens teilweise kondensiert.

Die Übertragung von Energie von BS1b auf den Strom im Sumpfverdampfer SV2a, bevorzugt die Beheizung des Stroms im Sumpfverdampfer SV2a durch BS1b erfolgt bevorzugt direkt. Direkte Übertragung bedeutet, dass BS1b und der Strom in SV2a sich zwar nicht direkt kontaktieren, aber dass Energie, insbesondere Wärme, von BS1b auf den Strom im SV2a übergeht, ohne dass ein zusätzliches Wärmeüberträgermedium vorhanden ist. Als Sumpfverdampfer SV2a können die dem Fachmann geläufigen Wärmeüberträger bzw. Wärmetauscher, insbesondere Verdampfer, eingesetzt werden.

Nachdem die Ströme VB1 und BS1b die Sumpfverdampfer SV1a respektive SV2a durchlaufen und Energie auf die jeweiligen Ströme übertragen haben werden diese Ströme VB1 und BS1b in Schritt (f) zumindest teilweise zur zweiten Destillationskolonne DK2 geleitet, wo dann die Auftrennung zwischen Isobutan und 1-Buten erfolgt, um einen möglichst reinen 1-Buten-Strom zu erhalten. Die beiden Ströme VB1 und BS1b können dabei unabhängig voneinander als unterschiedliche Zulaufströme oder gemeinsam zur zweiten Destillationskolonne DK2 geführt werden.

Bevor die Ströme zur zweiten Destillationskolonne in Schritt (f) zur zweiten Destillationskolonne geführt werden, werden die Ströme VB1 und BS1b nach einer bevorzugten Ausführungsform zum einem Flashbehälter geführt und dort entspannt, wodurch eine flüssige Phase FP1 der Ströme VB1 und BS1b anfällt. Der Flashbehälter kann zusätzlich einen Kondensator umfassen, um einen Teil der anfallenden gasförmigen Phase zu kondensieren.

In einer bevorzugten Ausführungsform der vorliegenden Erfindung werden die Ströme VB1 und BS1b gemeinsam zur zweiten Destillationskolonne geführt. Dazu werden die beiden Ströme insbesondere auf den gleichen Druck und auf die gleiche Temperatur gebracht. Sollen die Ströme VB1 und BS1b also gemeinsam zur Destillationskolonne geführt werden, ist es bevorzugt, dass die Ströme VB1 und BS1b im Flashbehälter vereint werden und als gemeinsame flüssige Phase FP1 anfallen.

Zumindest ein Teil FP1a der flüssigen Phase FP1 wird anschließend gemäß Schritt (f) zur Destillationskolonne DK2 geleitet. Dazu wird in einer besonders bevorzugten Ausführungsform eine Pumpe eingesetzt. Hier können dem Fachmann bekannte Pumpen eingesetzt werden. Geeignete Pumpen sind beispielsweise Chemie-Normpumpen.

Es ist weiterhin bevorzugt, dass ein von FP1a verschiedener Teil FP1b der flüssigen Phase FP1 als Rücklauf zur ersten Destillationskolonne DK1 zurückgeführt wird. Besonders bevorzugt ist es, dass dabei Energie vom Strom FP1b auf den Raffinat-2-Strom übertragen wird, bevor der Raffinat-2-Strom in die erste Destillationskolonne DK1 eingeleitet wird. Hierdurch wird der Raffinat-2-Strom vorgewärmt. Dies ist energetisch vorteilhaft, weil weniger Energie für die Trennaufgabe über die Sumpfverdampfer eingebracht werden muss. Die Übertragung von Energie von FP1b auf den Raffinat-2-Strom, bevorzugt die Beheizung des Raffinat-2-Stroms durch FP1b erfolgt bevorzugt direkt, d. h. ohne Einsatz eines (weiteren) Wärmeträgers. Dafür können dem Fachmann geläufige Wärmeüberträger bzw. Wärmetauscher eingesetzt werden.

In der zweiten Destillationskolonne DK2 werden die Ströme, die beide jeweils zumindest Isobutan und 1-Buten umfassen, in mindestens einen Brüdenstrom BS2, der zumindest Isobutan umfasst und am Kopf der DK2 entnommen wird, und in mindestens einen Produktstrom, der zumindest 1-Buten umfasst und am Sumpf der DK2 entnommen wird, aufgetrennt.

Als Destillationskolonne DK2 für die Auftrennung der beiden Ströme VB1 und BS1b kann jede beliebige, dem Fachmann bekannte Destillationskolonne eingesetzt werden. Bevorzugt enthält die Destillationskolonne DK2 Einbauten. Geeignete Einbauten sind zum Beispiel Böden, unstrukturierte Packungen (Füllkörper) oder strukturierte Packungen. Als Böden werden üblicherweise Glockenböden, Siebböden, Ventilböden, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel oder Intalox^{®}-Sättel verwendet. Strukturierte Packungen werden zum Beispiel unter dem Handelsnamen Mellapack^{®} der Firma Sulzer vertrieben. Neben den genannten Einbauten sind dem Fachmann weitere geeignete Einbauten bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Destillationskolonne DK2 möglichst gering bleibt und somit die mechanische Leistung des Verdichters und die Temperatur der zu verdampfenden beiden Ströme VB1 und BS1b gering bleibt.

In einer besonders bevorzugten Ausführungsform der vorliegenden Erfindung umfasst die zweite Destillationskolonne DK2 eine Vielzahl von Böden, vorzugsweise zwischen 150 und 300 Böden, weiterhin bevorzugt zwischen 170 und 220 Böden.

Die Entnahme mindestens einen Brüdenstroms BS2, der zumindest Isobutan umfasst, am Kopf der Destillationskolonne DK2 bedeutet im Rahmen der vorliegenden Erfindung insbesondere, dass der mindestens eine Brüdenstroms BS2 als Kopfstrom oder als Seitenabzug oberhalb der Einbauten in der Destillationskolonne DK2 entnommen wird.

Die Entnahme des mindestens einen Produktstroms, der zumindest 1-Buten umfasst, am Sumpf der Destillationskolonne DK2 bedeutet im Rahmen der vorliegenden Erfindung insbesondere, dass der mindestens eine Produktstrom direkt am Sumpf oder am unteren Boden der Destillationskolonne DK2 entnommen wird. Der Produktstrom enthält vorzugsweise mindestens 99 Gew.-% 1-Buten, weiterhin bevorzugt mindestens 99,5 Gew.-% 1-Buten, besonders bevorzugt mindestens 99,6 Gew.-% 1-Buten. Das 1-Buten ist das Zielprodukt des vorliegenden Verfahrens, weshalb der Produktstrom aus dem Verfahren ausgeschleust wird. Das 1-Buten kann beispielsweise als Co-Monomer bei der Herstellung von Polyethylen eingesetzt werden.

Hierbei gilt zu beachten, dass die Trennschärfe in der ersten Destillationskolonne DK1 letztlich die Reinheit vom 1-Buten im Produktstrom, der aus der zweiten Destillationskolonne DK2 entnommen wird, bestimmt. Butan wird nämlich in der DK2 ebenfalls als Schwersieder und deshalb mit dem 1-Buten anfallen. Dadurch würde das 1-Buten verunreinigt. Es sollte also darauf geachtet werden, dass die Auftrennung des Raffinat-2-Stroms in der DK1 möglichst so gefahren wird, dass kaum Butan zur DK2 gelangt. Deshalb ist es bevorzugt, dass der zur DK2 geführte Strom (1a, FP1a) maximal zwischen 500 und 900 ppm Butane enthält, bezogen auf die Gesamtmenge des Stroms.

Im Sumpf der zweiten Destillationskolonne DK2 liegt während des erfindungsgemäßen Verfahrens vorzugsweise eine Temperatur im Bereich von 30 bis 100 °C, vorzugsweise 45 bis 80 °C vor. Weiterhin bevorzugt liegt am Kopf der zweiten Destillationskolonne DK2 ein Druck im Bereich von 3 bis 12 bar absolut, vorzugsweise 5 bis 10 bar absolut vor.

Die Destillationskolonne DK2 kann weiterhin mit Rücklauf betrieben werden. Rücklauf bedeutet, dass der am oberen Ende der Destillationskolonne DK2 entnommene Brüdenstrom BS2 mindestens teilweise wieder der Destillationskolonne DK2 zugeführt wird. In den Fällen, in denen ein solcher Rücklauf eingestellt wird, beträgt das Rücklaufverhältnis dabei bevorzugt 10 bis 80, besonders bevorzugt 30 bis 50.

Ein Rücklauf kann dadurch eingestellt werden, dass am Kopf der Destillationskolonne DK2 ein Kondensator angebracht wird. In dem Kondensator wird der Brüdenstrom BS2teilweise kondensiert und der Destillationskolonne DK2 wieder zugeführt. Unter einem Rücklaufverhältnis wird allgemein und im Sinne dieser Erfindung das Verhältnis aus dem Anteil des aus der Kolonne entnommenen Massenstroms (kg/h), der wieder auf die Kolonne in flüssiger Form zurückgeführt wird (Rücklauf) zu dem Anteil dieses Massenstroms (kg/h), der in flüssiger Form oder gasförmiger Form von der jeweiligen Kolonne abgeführt wird, verstanden.

Anschließend wird in Schritt (g) ein der Brüdenstroms BS2 zumindest teilweise verdichtet, wodurch ein gegenüber dem Brüdenstrom BS2 verdichteter Strom VB2 entsteht. Der Druck von VB2 ist nach dem Verdichten höher als der Druck von BS2. Der genaue Wert für den Druck von VB2 kann in Abhängigkeit der Anforderungen bei der nachfolgenden Energieübertragung vom Fachmann eingestellt werden, solange die Bedingung Druck VB2 > Druck BS2 erfüllt ist. Der Quotient aus Druck VB2 / Druck BS2 (Drücke jeweils in bar abs.) liegt bevorzugt im Bereich von 1,1 bis 10, bevorzugter 1,2 bis 8, bevorzugter 1,25 bis 7, am bevorzugtesten 1,3 bis 6. Es kann vorteilhaft sein den Strom BS2 vor dem Verdichten zu erwärmen, damit bei der Verdichtung kein zweiphasiges Gemisch entsteht. Die Erwärmung kann mittels interner (s. WT-2 in Fig. 3 und 4 der vorliegenden Anmeldung) oder externer Wärmequellen durchgeführt werden.

Die Temperatur des Teilstroms VB2 ist vorzugsweise höher als die Temperatur des Brüdenstroms BS2, und der Quotient aus Temperatur VB2 / Temperatur BS2 (Temperatur jeweils in K) liegt bevorzugt im Bereich von 1,03 bis 10, bevorzugter 1,04 bis 9, bevorzugter 1,05 bis 8, bevorzugter 1,06 bis 7, bevorzugter 1,07 bis 6, am bevorzugtesten 1,08 bis 5.

Das Verdichten des mindestens einen Teils des Brüdenstroms BS2 in Schritt (c) kann auf jede beliebige, dem Fachmann bekannte Art erfolgen. So kann die Verdichtung zum Beispiel mechanisch und einstufig oder mehrstufig durchgeführt werden. Bei einer mehrstufigen Verdichtung können mehrere Verdichter gleicher Bauart oder Verdichter unterschiedlicher Bauart eingesetzt werden. Eine mehrstufige Verdichtung kann mit einer oder mehreren Verdichtermaschinen erfolgen. Der Einsatz einer einstufigen Verdichtung oder einer mehrstufigen Verdichtung ist davon abhängig vom Verdichtungsverhältnis und somit, auf welchen Druck der Brüdenteilstrom BS2 verdichtet werden soll.

Als Verdichter im erfindungsgemäßen Verfahren, insbesondere zum Verdichten des Brüdenstroms B2 zu VB2, eignet sich jeder beliebige, dem Fachmann bekannte Verdichter, bevorzugt mechanische Verdichter, mit dem sich Gasströme verdichten lassen. Geeignete Verdichter sind zum Beispiel ein- oder mehrstufige Turbinen, Kolbenverdichter, Schraubenverdichter, Zentrifugalverdichter oder Axialverdichter.

Im Schritt (h) des erfindungsgemäßen Verfahrens wird Energie vom verdichteten Strom VB2 auf den Strom im Sumpfverdampfer SV1b übertragen. Durch den Schritt (d) sinkt die Energie von VB2, so dass VB2 insbesondere mindestens teilweise kondensiert. Die Phrase Übertragung von Energie bedeutet erfindungsgemäß insbesondere Beheizung, also Übertragung von Energie in Form von Wärme.

Die Übertragung von Energie von VB2 auf den Strom im Sumpfverdampfer SV1b, bevorzugt die Beheizung des Stroms im Sumpfverdampfer SV1b durch VB2 erfolgt bevorzugt direkt. Direkte Übertragung bedeutet, dass VB2 und der Strom in SV1b sich zwar nicht direkt kontaktieren, aber dass Energie, insbesondere Wärme, von VB2 auf den Strom in SV1b übergeht, ohne dass ein zusätzliches Wärmeüberträgermedium vorhanden ist. Als Sumpfverdampfer SV1b können die dem Fachmann geläufigen Wärmeüberträger bzw. Wärmetauscher, insbesondere Verdampfer, eingesetzt werden.

Nachdem der Strom VB2 den Sumpfverdampfer SV1b durchlaufen und Energie auf den dort vorhandenen Strom übertragen hat, kann VB2 als Isobutan-Strom IB1 aus dem Verfahren ausgeschleust werden. Bevor VB2 aber als IB1 aus dem Verfahren abgeführt wird, wird der Strom VB2 nach einer bevorzugten Ausführungsform der vorliegenden Erfindung zum einem Flashbehälter geführt und dort entspannt, wodurch eine flüssige Phase FP2 anfällt. Der Flashbehälter kann zusätzlich einen Kondensator umfassen, um einen Teil der anfallenden gasförmigen Phase zu kondensieren.

Zumindest ein Teil FP2a der flüssigen Phase FP2 kann anschließend als Isobutan-Strom IB1 aus dem Verfahren ausgeschleust werden. Dazu wird in einer besonders bevorzugten Ausführungsform eine Pumpe eingesetzt. Unter Umständen und bei ausreichendem Druckverhältnis wäre es auch möglich, dass keine Pumpe eingesetzt wird. Sofern eine Pumpe eingesetzt wird, können dem Fachmann bekannte Pumpen eingesetzt werden. Geeignete Pumpen sind beispielsweise Chemie-Normpumpen. Es ist weiterhin bevorzugt, dass ein von FP2a verschiedener Teil FP2b der flüssigen Phase FP1 als Rücklauf zur ersten Destillationskolonne DK2 zurückgeführt wird.

In der grundsätzlichen Ausgestaltung der vorliegenden Erfindung werden die beiden Ströme BS1a und BS2 jeweils mit einem einzigen Verdichter verdichtet. In einer bevorzugten Ausführungsform der vorliegenden Erfindung werden die beiden Ströme BS1a und BS2 in einem einzigen, vorzugsweise mehrstufigen Verdichter verdichtet. Die Anzahl der notwendigen Stufen hängt davon ab, welches Verdichtungsverhältnis angestrebt wird.

Die Wärmeintegration durch die hier beschriebene Brüdenverdichtung ließe sich auch mit anderen Maßnahmen zur Wärmeintegration kombinieren. Möglich wäre es hier noch eine oder mehrere Wärmepumpe(n) vorzusehen.

Die vorliegende Erfindung wird nachfolgend anhand von Abbildungen erläutert. Die Abbildungen dienen der Veranschaulichung, sind aber nicht einschränkend zu verstehen.

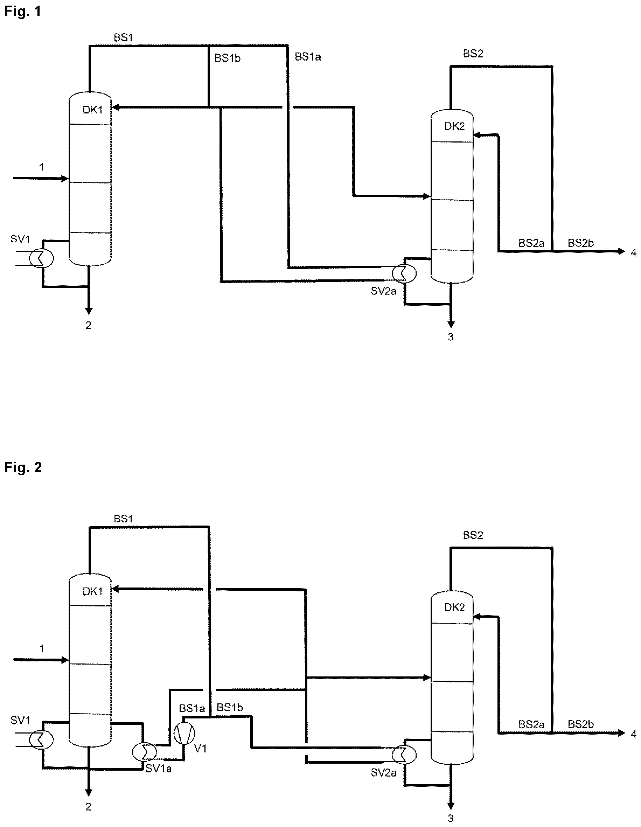

Fig. 1 zeigt eine Ausgestaltung nach dem Stand der Technik. Der Raffinat-2-Strom (1) wird zur ersten Destillationskolonne DK1 geführt und dort in einen Brüdenstrom BS1, der zumindest 1-Buten und Isobutan umfasst und am Kopf der DK1 entnommen wird, und in einen Sumpfstrom (2), der zumindest 1-Buten und 2-Buten umfasst und am Sumpf der DK1 entnommen wird. Dieser Strom (2) kann zu einer Oligomerisierung (nicht eingezeichnet) geführt werden. Der Brüdenstrom BS1 wird in die beiden Brüdenströme BS1a und BS1b aufgetrennt, ggf. nach vorheriger Kondensation (nicht eingezeichnet). BS1a wird als Rücklauf zur ersten Destillationskolonne DK1 geführt. BS1b wird ohne zusätzliche Verdichtung zum Sumpfverdampfer SV2a der zweiten Destillationskolonne geleitet und überträgt dort Energie auf den dort vorhandenen Strom, der aus dem Sumpf kommend erhitzt und dann wieder zurückgeführt wird. Dies entspricht einer klassischen Zweidruckschaltung, bei der die DK1 mit einem höheren Druck betrieben wird als die DK2 (siehe auch Beispiel 1). In der Destillationskolonne 2 werden die Ströme in einen Brüdenstrom BS2, der zumindest Isobutan umfasst und am Kopf der DK2 entnommen wird, und in einen Produktstrom (3), der zumindest 1-Buten umfasst und am Sumpf der DK2 entnommen wird, aufgetrennt. Der Brüdenstrom wird nach optionaler Kondensation in die Ströme B2a und B2b aufgetrennt. B2a geht als Rücklauf zurück zur DK2 und B2b wird als Isobutan-Strom (4) aus dem Verfahren ausgeschleust.

Fig. 2 zeigt eine ebenfalls nicht erfindungsgemäße Ausgestaltung, die weitestgehend der Ausgestaltung nach Fig. 1 entspricht. Der einzige Unterschied besteht darin, dass der Strom BS1a mit dem Verdichter V1 auf einen höheren Druck gebracht und anschließend zur Energieübertragung zum Sumpfverdampfer SV1a geleitet wird. Die beiden Ströme BS1a und BS1b werden nach der Energieübertragung kombiniert und gehen teilweise als Rücklauf zur DK1 und teilweise als Feed zur DK2.

Fig. 3 zeigt eine erfindungsgemäße Ausführungsform, die in weiten Teilen mit der in Fig. 2 dargestellten Ausführungsform identisch ist. Der Unterschied besteht darin, dass der mit dem Verdichter V1 verdichtete Strom VB1 genannt wird. Zusätzlich dazu wird der Brüdenstrom BS2 mittels eines weiteren Verdichters V2 verdichtet und als VB2 zum Sumpfverdampfer SV1b geführt. Auf diese Weise kann auf den Einsatz von externem Heizdampf vollständig verzichtet werden.

Fig. 4 zeigt eine ebenfalls erfindungsgemäße Ausführungsform, die in weiten Teilen der Fig. 3 entspricht. Der Unterschied besteht darin, dass die beiden Ströme VB1 und BS1b nach der Energieübertragung in den jeweiligen Sumpfverdampfern SV1a und SV2a zu einem Flashbehälter gefahren werden, wo durch die Entspannung eine flüssige Phase FP1 anfällt. Diese flüssige Phase wird in die beiden Ströme FP1a, der zur zweiten Destillationskolonne DK2 geführt wird, und FP1b, der als Rücklauf zur ersten Destillationskolonne geleitet wird, aufgetrennt. Vergleichbares passiert mit dem Strom VB2 nach der Energieübertragung im Sumpfverdampfer SV1b. VB1 landet danach in einem Flashbehälter und kondensiert zumindest teilweise als flüssige Phase FP2, von der ein Teil FP2a aus Isobutan-Strom (4) das Verfahren verlässt und ein anderer Teil FP2b als Rücklauf zur zweiten Destillationskolonne zurückgeführt wird.

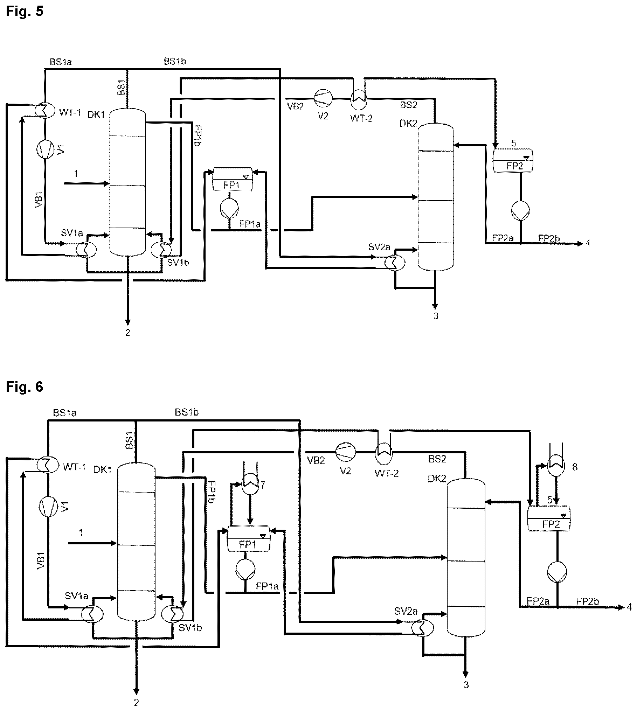

Fig. 5 zeigt eine Ausführungsform, die in weiten Teilen mit den in Fig. 1 und in Fig. 2 dargestellten Ausführungsformen identisch ist. Der Unterschied besteht darin, dass zwei zusätzliche Wärmetauscher (WT-1, WT-2) vorhanden sind, über die Energie von den Strömen VB1 (in WT-1) und VB2 (in WT-2) auf die Ströme BS1a (in WT-1) und BS2 (in WT-2) übertragen wird, Hierdurch kann zusätzlich Energie eingespart werden.

Fig. 6 zeigt eine Ausführungsform, die in weiten Teilen mit den in Fig. 1 bis Fig. 3 dargestellten Ausführungsformen identisch ist. Der Unterschied besteht darin, dass auf den beiden Flashbehältern jeweils ein Kondensator (7, 8) zur Regelung des Drucks installiert ist. Zusätzlich kann der Kondensator auch für den Anfahrprozess verwendet werden.

### Beispiele

Für alle nachfolgenden Beispiele wurde ein Raffinat 2-Strom von 55t/h eingesetzt. Der Raffinat-2-Stroms hat die folgende Zusammensetzung: 1-Buten 45,1 % / n-Butan 22,4% /trans-2-Buten 15,9% / cis-2-Buten 8,9% / Isobutan 7,4% / Isobuten 45 ppm und Wasser 590 ppm.

Die für den Betrieb, der in den Beispielen dargestellten Anlagen zur Abtrennung von 1-Buten aus Raffinat 2 notwendige Menge an Energie wurde anhand einer Simulation mittels Aspen V10 errechnet. Die Stoffdaten wurden durch Betriebsdaten und Betriebsversuche validiert.

### Beispiel 1 (nicht erfindungsgemäß):

In der Ausführungsform nach Fig. 1 wird ausschließlich eine direkte Wärmeintegration zwischen der Kolonne DK1 und DK2 durchgeführt (wie in DE 10 2005 062 700 A1 offenbart). Hierbei wird die Kolonne DK1 bei einem Kopfdruck von 11 bar und die Kolonne DK2 von 7 bar betrieben. So kann mit einem Teil des Brüdenstroms BS1b über den Sumpfverdampfer SV2a an der DK2 Wärme übertragen werden. Die Wärmemenge, die für eine 1-Butenmenge von 10,3 t/h bei einer Reinheit von 99,6% 1-Buten erforderlich ist, beträgt 10,3 MW in der DK2. Hierzu wird ein Brüdenstrom von 125,5 t/h der DK1 verwendet, um die DK2 zu beheizen. Nichtsdestotrotz müssen an der DK1 15,9 MW über eine externe Wärmequelle/Medium (z. B. Heizdampf) eingetragen werden.

### Beispiel 2 (nicht erfindungsgemäß):

In dieser Ausführungsform wird eine direkte Wärmeintegration zwischen der Kolonne DK1 und DK2 durchgeführt (wie in DE 10 2005 062 700 A1 offenbart) und um eine Brüdenverdichtung ergänzt (s. Fig. 2). Die Kondensationswärme von 4,0 MW der DK1, die im Beispiel 1 nicht für die Wärmeintegration zwischen DK1 und DK2 verwendet wird, wird über eine einstufige Brüdenverdichtung über Verdichter V1 nutzbar gemacht und über einen zweiten Sumpfverdampfer SV1a an der DK1 eingetragen. Insgesamt können 4,4 MW durch die Brüdenverdichtung über den Sumpfverdampfer SV1a übertragen werden.

Demzufolge wird der externe Heizbedarf an der Kolonne DK1 von 15,9 MW (Beispiel 1) auf 11,5 MW reduziert. In Summe werden müssen 382 kW elektrische Leistung an dem Verdichter V1 aufgebracht werden, um den Brüden von 11 bar auf 16,9 bar zu verdichten, um so eine treibende Temperaturdifferenz am SV1a von 8 K zu erzeugen.

### Beispiel 3 (erfindungsgemäß):

In dieser Ausführungsform wird eine direkte Wärmeintegration zwischen der Kolonne DK1 und DK2 durchgeführt (wie in DE 10 2005 062 700 A1 offenbart) und erfindungsgemäß um eine mehrstufige Brüdenverdichtung ergänzt (s. Fig. 3). Die Ausführungsform, die in Beispiel 2 beschrieben wird, wird um eine weitere Brüdenverdichtung ergänzt. Der Brüden der DK2 wird verdichtet, um die Kondensationswärme von 9,9 MW der DK2 für die DK1 nutzbar zu machen. Die über den Verdichter V2 aufgewertete Kondensationswärme wird über einen weiteren Sumpfverdampfer SV1b übertragen. Hierzu wird der Brüdenstrom der zweiten Kolonne DK2 von 7 bar auf 21 bar verdichtet. Es ist eine elektrische Leistung von 2,6 MW erforderlich. Insgesamt können 11,5 MW über den SV1b übertragen werden, sodass keine externe Wärmequelle für den stationären Betrieb erforderlich ist. Demzufolge wurde der energieintensive Prozess der 1-Butendestillation vollständig verstromt.

Nachfolgend sie die Ergebnisse der Beispiele 1 bis 3 in der Tabelle 1 zusammengefasst.

**Tabelle 1: Zusammenfassung Beispiele**

| | Beispiel 1 | Beispiel 2 | Beispiel 3* |
|---|---|---|---|
| Externe Heizleistung [MW] | 15,9 | 11,5 | 0 |
| Elektrische Leistung Verdichter [MW] | 0 | 0,38 | 2,98 |

| | | | |
|---|---|---|---|
| * Erfindungsgemäß | | | |

Es zeigt sich, dass die erfindungsgemäße Ausgestaltung des Verfahrens dazu führt, dass deutlich weniger externe Heizleistung eingesetzt werden muss als bei der bekannten Lösung. Das Einsparpotential ist also erheblich. Die dazu zusätzlich erforderliche elektrische Leistung zum Betrieb der Verdichter ist deutlich geringer und kann bei Einsatz von grünem Strom einen CO₂-neutralen Betrieb ermöglichen.

## Patentansprüche

1. Verfahren zur Abtrennung von 1-Buten aus einem Kohlenwasserstoffstrom, der zumindest 1-Buten, 2-Buten, n-Butan und Isobutan enthält, in einer Abtrenneinheit, die mindestens zwei Destillationskolonnen DK1 und DK2 umfasst, wobei
die erste Destillationskolonne DK1 mindestens zwei Sumpfverdampfer SV1a und SV1b und die zweite Destillationskolonne DK2 mindestens einen Sumpfverdampfer SV2a aufweist;
die Sumpfverdampfer SV1a und SV1b jeweils mit einem Strom gespeist werden, der am unteren Ende der DK1 entnommen wird und nach Durchlaufen des jeweiligen Sumpfverdampfers wieder zur DK1 geführt wird;
der Sumpfverdampfer SV2a mit einem Strom gespeist wird, der am unteren Ende der DK2 entnommen wird und nach Durchlaufen des jeweiligen Sumpfverdampfers wieder zur DK2 geführt wird; wobei das Verfahren die folgenden Schritte umfasst
(a) der Raffinat-2-Strom zur ersten Destillationskolonne DK1 geleitet wird und in der DK1 in mindestens einen Brüdenstrom BS1, der zumindest 1-Buten und Isobutan umfasst und am Kopf der DK1 entnommen wird, und in mindestens einen Sumpfstrom, der zumindest 1-Buten und 2-Buten umfasst und am Sumpf der DK1 entnommen wird, aufgetrennt wird;
(b) der Brüdenstrom in mindestens zwei Teilströme BS1a und BS1b aufgetrennt wird;
(c) ein erster Teil BS1a des Brüdenstroms BS1 verdichtet wird, wodurch ein gegenüber dem Brüdenstrom BS1a verdichteter Strom VB1 entsteht;
(d) Energie vom verdichteten Strom VB1 auf den Strom im Sumpfverdampfer SV1a übertragen wird;
(e) ein von BS1a verschiedener Teil BS1b des Brüdenstroms BS1 zum Sumpfverdampfer SV2a geführt wird und dort Energie von BS1b auf den Strom im Sumpfverdampfer SV2a übertragen wird;
(f) die Ströme VB1 und BS1b zumindest teilweise zur zweiten Destillationskolonne DK2 geleitet werden und in der DK2 in mindestens einen Brüdenstrom BS2, der zumindest Isobutan umfasst und am Kopf der DK2 entnommen wird, und in mindestens einen Produktstrom, der zumindest 1-Buten umfasst und am Sumpf der DK2 entnommen wird, aufgetrennt wird;
(g) der Brüdenstroms BS2 zumindest teilweise verdichtet wird, wodurch ein gegenüber dem Brüdenstrom BS2 verdichteter Strom VB2 entsteht; und
(h) Energie vom verdichteten Strom VB2 auf den Strom im Sumpfverdampfer SV1b übertragen wird.

2. Verfahren nach Anspruch 1, wobei die Ströme VB1 und BS1b zum einem Flashbehälter geführt und dort entspannt werden, wodurch eine flüssige Phase FP1 der Brüdenströme BS1a und BS1b anfällt.

3. Verfahren nach Anspruch 2, wobei die Ströme VB1 und BS1b im Flashbehälter vereint werden und als gemeinsame flüssige Phase FP1 anfallen.

4. Verfahren nach Anspruch 3, wobei zumindest ein Teil FP1a der flüssigen Phase FP1 zur Destillationskolonne DK2 geleitet wird, vorzugsweise mittels einer Pumpe

5. Verfahren nach Anspruch 3 oder 4, wobei ein anderer Teil FP1b der flüssigen Phase FP1 als Rücklauf zur Destillationskolonne DK1 zurückgeführt wird.

6. Verfahren nach Anspruch 5, wobei Energie vom Strom FP1b auf den Raffinat-2-Strom übertragen wird, bevor der Raffinat-2-Strom in die erste Destillationskolonne DK1 eingeleitet wird.

7. Verfahren nach einem der vorhergehenden Ansprüche, wobei im Sumpf der ersten Destillationskolonne DK1 eine Temperatur im Bereich von 40 bis 110 °C, vorzugsweise 50 bis 100 °C vorliegt.

8. Verfahren nach einem der vorhergehenden Ansprüche, wobei im Sumpf der zweiten Destillationskolonne DK2 eine Temperatur im Bereich von 30 bis 100 °C, vorzugsweise 45 bis 80 °C vorliegt.

9. Verfahren nach einem der vorhergehenden Ansprüche, wobei die Destillationskolonne DK1 zwischen 150 und 300 Böden aufweist.

10. Verfahren nach einem der vorhergehenden Ansprüche, wobei die Destillationskolonne DK2 zwischen 150 und 300 Böden aufweist.

11. Verfahren einem der vorhergehenden Ansprüche, wobei für die Verdichtung der Ströme BS1a und BS2 nur ein einziger Verdichter eingesetzt wird.

12. Verfahren nach einem der vorhergehenden Ansprüche, wobei der Strom VB2 in einen Flashbehälter entspannt wird, wo eine flüssige Phase FP2 anfällt.

13. Verfahren nach Anspruch 12, wobei zumindest ein Teil FP2a der flüssigen Phase FP2 als Produktstrom aus dem Verfahren ausgeschleust wird, vorzugsweise mittels einer Pumpe

14. Verfahren nach Anspruch 13, wobei ein anderer Teil FP2b der flüssigen Phase FP2 als Rücklauf zur Destillationskolonne DK2 zurückgeführt wird.

15. Verfahren nach einem der vorhergehenden Ansprüche, wobei der Produktstrom mindestens 99 Gew.-% 1-Buten, vorzugsweise mindestens 99,5 Gew.-% 1-Buten, besonders bevorzugt mindestens 99,6 Gew.-% 1-Buten enthält.
